# EUROPÄISCHE PATENTANMELDUNG

(11) **EP 2 606 926 A1**
(43) Veröffentlichungstag der Anmeldung: **26.06.2013**
(21) Anmeldenummer: 11075274.8
(22) Anmeldetag: 22.12.2011
(51) Int. Cl.: A61M 5/24, A61M 5/315

(54) **Mehrkammermischbehälter**

(71) Anmelder: B. Braun Melsungen AG, 34212 Melsungen (DE)
(72) Erfinder: Harand, Ralf, 34302 Guxhagen (DE); Krüger, Volker, 34329 Nieste (DE); Beine, Joachim, 34302 Guxhagen (DE); Vonhof, Sandra, 34212 Melsungen (DE)
(74) Vertreter: Arth, Hans-Lothar

(57) **Zusammenfassung**

Die Erfindung betrifft einen Mehrkammermischbehälter bestehend aus Stülpbehälter, Entnahmebehälter und verschiedenen optional vorhanden Zwischenbehältern, weiterhin eine Vorrichtung zur Aufnahme und Anwendung des Mehrkammermischbehälters und Methoden zum Mischen von Flüssigkeiten oder Flüssigkeiten und Feststoffen unter aseptischen Bedingungen in dem Mehrkammermischbehälter.

## Beschreibung

Die Erfindung betrifft einen Mehrkammermischbehälter bestehend aus Stülpbehälter, Entnahmebehälter und verschiedenen optional vorhanden Zwischenbehältern, weiterhin eine Vorrichtung zur Aufnahme und Anwendung des Mehrkammermischbehälters und Methoden zum Mischen von Flüssigkeiten oder Flüssigkeiten und Feststoffen unter aseptischen Bedingungen in dem Mehrkammermischbehälter.

### Hintergrund

Mehrkammermischsysteme dienen zum Vermischen von zwei oder mehreren Komponenten, vor allem von solchen, die nur kurzfristig miteinander in Kontakt sein sollen. Viele Einzelkomponenten sind in Lösung oder Mischung mit anderen Komponenten instabil, so dass es vorteilhaft ist, die Komponenten getrennt zu lagern und nur kurz vor der Verwendung der Komponenten miteinander in Mischung oder Lösung zu bringen. Diese betrifft z.B. Lösungen für medizinische Verwendungen, aber auch in industriellen Anwendungen wir der Farbauftragung, Mischung von Chemikalien oder in der Materialherstellung.

In vielen medizinischen, zahnmedizinischen und veterinärmedizinischen ist es notwendig, erst kurz vor der Verwendung bzw. Verabreichung die Komponenten einer Lösung miteinander zu mischen. Diese Präparate und Zubereitungen können flüssige Komponenten aber auch feste Komponenten, z.B. Pulver, umfassen. Viele Wirkstoffe sind verdünnter Lösung oder überhaupt in gelöster Form nicht stabil, so dass die Mischung und Verabreichung von Lösungen in kurzer Zeit sehr erwünscht ist.Für die Mischung von Flüssigkeiten und/oder Feststoffen sind verschiedene Systeme bekannt. So sind im Stand der Technik vor allem Spritzen-artige Behälter zum Mischen von zwei oder mehr Komponenten bekannt, wobei die meisten Systeme Zweikammermischsysteme sind. Dabei werden die meisten Systeme industriell vorgefertigt. In US2007/0185438 ist eine Vorrichtung offenbart, in der in einer Ampulle mit mehreren Kammern die einzelnen Komponenten einer Mehrkomponentenmischung in verschiedenen Kammern aufbewahrt werden. Für die Mischung der Komponenten werden die Behälter mit einem zur Vorrichtung gehörenden Kolbenelement durchstoßen. Damit wird aus mehreren Kammern ein einiges Volumen. Die Ampulle kann eine Spritzen-artige Vorrichtung gesetzt werden und unter Verwendung einer angebrachten Nadel direkt als Spritze zur Verabreichung der Mischung verwendet werden. Die Komponenten sind hier durch die Vorfertigung in der Ampulle festgelegt und nicht frei miteinander kombinierbar. Die Anmeldung US2010/0122310 betrifft eine Mehrkammerkartusche, in der entlang der Längsachse zwei oder mehrere Kammern angeordnet sind, und zu der weiterhin zwei Kolben gehören, mit deren Hilfe die Komponenten gemischt und das Gemisch durch eine Düse auch verwendet werden kann.

Die offenbarten Vorrichtungen sind jeweils vorgefertigt und an das Vorhandensein von Spritzen-artigen Kartuschensystemen gebunden. Dabei sind die Kompartimente innerhalb einer vorgefertigten Ampulle oder Kartusche oder Spritze o. ä. durch Trennung durch Membranen oder feste Wände getrennt, jedoch sind nach Herstellung und Befüllung der Kompartimente durch den Hersteller keine Variation der einzelnen Komponenten, also beispielsweise Austausch oder Veränderungen der Reihenfolge in der Anordnung möglich. Weiterhin ist die Anzahl der Kompartimente im Stand der Technik einmal vorgefertigt nicht mehr variierbar. Ein weiterer Nachteil der Ausführungsformen des Standes der Technik ist, dass immer Nadel- und Kolbenartige Teile feste Bestandteile der Vorrichtung sind, die zum Durchstechen und Mischen erforderlich werden.

Aufgabe der vorliegenden Patentanmeldung ist es, eine Vorrichtung bereitzustellen, die zur Mischung von mindestens zwei Komponenten geeignet ist, wobei die Komponenten und auch die Zahl der Komponenten durch Bereitstellung in verschiedenen Behältern variierbar sein sollen, und die ferner so ausgestaltet sein sollen, dass die Implementierung von Nadeln und Kolben in der Vorrichtung zum Durchmischen der Lösungen nicht notwendig ist.

Diese Aufgabe wird durch die Bereitstellung eines Stülpbehälters gemäß Anspruch 1, der Mehrkammermischbehälter gemäß Anspruch 6, 7 oder 8, der Vorrichtung gemäß Anspruch 10 und der Methode gemäß Anspruch 13 gelöst. Weitere vorteilhafte Ausgestaltungen, Aspekte und Details der Erfindung ergeben sich aus den abhängigen Ansprüchen, der Beschreibung, den Beispielen und den Figuren.

### Beschreibung der Erfindung

Überraschend wurde gefunden, dass der erfindungsgemäße Mehrkammermischbehälter zur Bereitstellung und Mischung der Komponenten die gestellte Aufgabe löst. Der erfindungsgemäße Mehrkammermischbehälter dient zur getrennten Lagerung von mindestens einem Lösungsmittel und einem Wirkstoff, der beispielsweise im lyophilisierten, sprühgetrockneten oder gefriergetrockneten Zustand stabiler als im gelösten Zustand ist, so dass eine getrennte Lagerung von Lösungsmittel und Wirkstoff im Mehrkammermischbehälter erfolgen kann und die aseptische Durchmischung kurz vor der Anwendung ebenfalls in dem erfindungsgemäßen Mehrkammermischbehälter durchgeführt werden kann.

Zu den Wirkstoffen, welche vorzugsweise für parenterale Anwendungen in dem Mehrkammermischbehälter aufbewahrt werden können, zählen beispielsweise Antibiotika, Analgetika, anti-entzündliche Wirkstoffe, Steroide, antiproliferativen, immunsuppressive, fungizide, zytostatische, antimigrative, antiphlogistische, zytotoxische, antiangiogene oder/und antithrombotische Wirkstoffe. Flüssigkeiten zur Lösung oder Mischung umfassen aqua bidest., isotonische Natriumchloridlösung, oder verschiedene andere isotonische Salzlösungen.

Der erfindungsgemäße Mehrkammermischbehälter besteht in der einfachsten Ausführungsform aus dem Stülpbehälter und einem Entnahmebehälter, welche aseptisch miteinander verbunden sind. Das Lösungsmittel befindet sich in dem Stülpbehälter und der oder die Wirkstoffe in dem Entnahmebehälter. Der Wirkstoff oder die Wirkstoffkombination in dem Entnahmebehälter ist vorzugsweise ein Feststoff, der sich als Feststoff länger lagern lässt als in Lösung, so dass die Wirkstofflösung erst kurz vor der Anwendung hergestellt werden soll.

Ist es notwendig, mehrer Wirkstoffe oder mehrere Lösungsmittel oder einen flüssigen Wirkstoff und einen festen Wirkstoff getrennt vom Lösungsmittel zu lagern, so kann der erfindungsgemäße Mehrkammermischbehälter neben dem Stülpbehälter und dem Entnahmebehälter auch noch einen, zwei oder mehrere Zwischenbehälter aufweisen, welche sich zwischen dem Stülpbehälter und dem Entnahmebehälter befinden und vorzugsweise zur Aufnahme jeweils einer Komponente dienen.

Stülpbehälter, Entnahmebehälter und Zwischenbehälter werden generisch auch als Komponentenbehälter bezeichnet.

Die Komponentenbehälter sind verschlossen, wobei der Verschluss der Komponentenbehälter vorzugsweise durch eine Versiegelung mit einer Siegelfolie geschieht. Zweck der Komponentenbehälter ist es, den Inhalt steril und keimfrei zu halten. Die erfindungsgemäßen Komponentenbehälter weisen weiterhin vorzugsweise mindestens eine Zentriernut, Vertiefung oder auch einen Zapfen in der Seitenfläche auf. Diese sind vorteilhaft, weil bei einer Anordnung des oder der Komponentenbehälter in einer Vorrichtung zur Durchmischung ein mechanischer Halt des oder der Behälter gewährleistet werden kann.

Es werden also alle Komponentenbehälter, d.h. zwei (Bi-System), drei (Tri-System), vier (Quad-System) oder ganz allgemein mehrere Komponentenbehälter (Multisystem) formschlüssig, kraftschlüssig oder stoffschlüssig und aseptisch miteinander verbunden. Die Gestaltung der Container erlaubt ausserdem das Verbinden mehrerer Komponentenbehälter zu einem Bi-, Tri- oder Quad-System, um mehrere über einen längeren Zeitraum inkompatible Inhaltsstoffe getrennt zu lagern und kurz vor der Anwendung miteinander zu mischen.

Ein Bi-System besteht dabei aus einem Stülpbehälter und einem Entnahmebehälter, ein Tri-System aus einem Stülpbehälter, einem Zwischenbehälter und einem Entnahmebehälter und ein Quad-System aus einem Stülpbehälter, einem ersten Zwischenbehälter, einem zweiten Zwischenbehälter und einem Entnahmebehälter. Jeder Komponentenbehälter kann dabei eine Komponente oder auch ein Komponentengemisch der später herzustellenden Lösung aufnehmen, getrennt von den anderen Komponenten aufbewahren sowie steril und keimfrei halten. Auch die Verbindung der Komponentenbehälter miteinander erfolgt aseptisch.

Optional können die Komponentenbehälter jeweils einen Fügerand aufweisen, der das Zusammenfügen eines Komponentenbehälters mit einem oder zwei weiteren Komponentenbehältern ermöglicht, der Verbindung einen mechanischen halt vermittelt, und den Verschluss der Verbindung nach außen erleichtert.

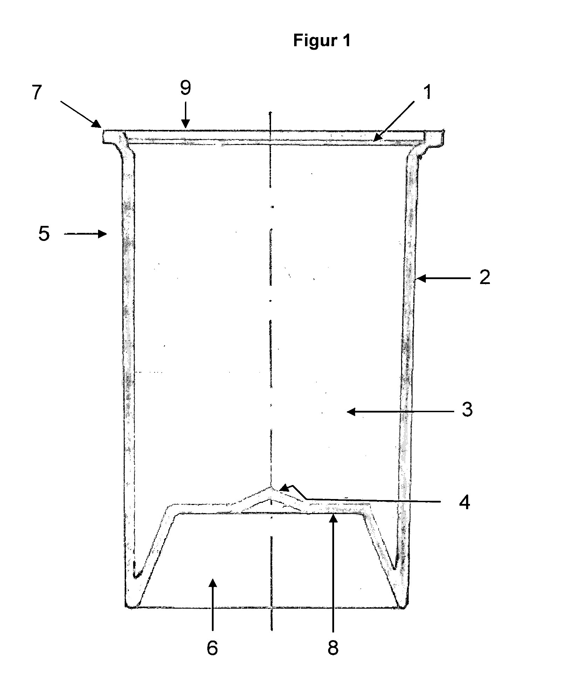

Zur Erfindung gehören verschiedene Ausführungsformen der Komponentenbehälter. Der Stülpbehälter ist vorzugsweise ein Flüssigkeitsbehälter. Ein erfindungsgemäßer Stülpbehälter weist eine stülpbare Seitenwandung, eine versiegelbare Stirnseite, weiterhin vorzugsweise eine innenliegende Nase oder Wölbung zum Durchdrücken der Siegelfolie, die durch Umstülpen der Seitenwandung axial bewegt werden kann, und vorzugsweise mindestens eine Zentriernut oder Vertiefung in der Wandung auf (Figur 1). Die stülpbare Seitenwandung ist vorteilhaft, weil dadurch ein Eindrücken einer Behälterfläche durch Druck von außen ermöglicht wird. Die einstülpbaren Teile der Seitenwandung sind vorzugsweise weicher, also schwächer in der Materialbeschaffenheit, um ein leichtes Eindrücken zu ermöglichen, ohne dass das Material beschädigt wird oder ein zu hoher Kraftaufwand notwendig wird. Das Eindrücken bzw. Einstülpen wird dabei bevorzugt von sogenannten Rastlinien unterstützt, um ein leichteres Eindrücken zu ermöglichen. Die Rastlinien zeichnen sich durch einen Übergang zu einer stärkeren oder schwächeren Materialbeschaffenheit aus, die die Stülpeigenschaften verändert.

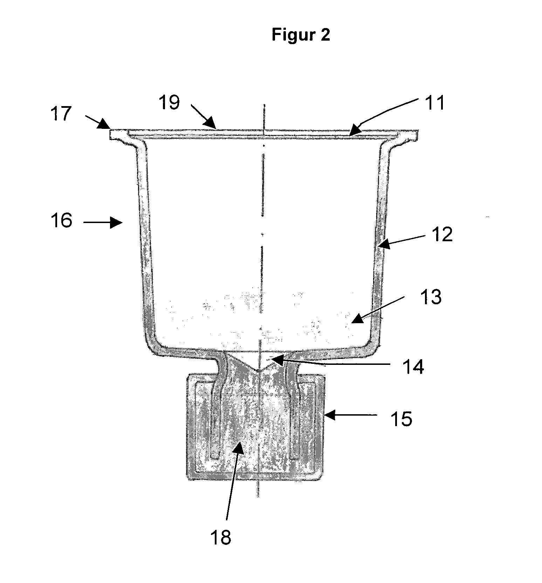

Ein weiterer erfindungsgemäßer Behälter ist der Entnahmebehälter. Der Entnahmebehälter wird in seiner äußeren Form durch Stirnseite, Seitenwandung und Verschlussseite charakterisiert. Die Stirnseite ist wie beim Stülpbehälter durch eine durchdrückbare Folie versiegelt. Der Entnahmebehälter kann dabei eine Flüssigkeit oder einen festen Stoff enthalten. Feste Stoffe sind bevorzugt pulverförmig, ebenfalls bevorzugt lyophilisiert und weiterhin bevorzugt gefriergetrocknet. Die Form des Entnahmebehälters ist dabei vorzugsweise der des Stülpbehälters angepasst, d.h. der Durchmesser und die Form der Stirnseite entspricht der des Stülpbehälters, wodurch eine dichte, also stoffschlüssige Verbindung zwischen beiden Komponentenbehältern gewährleistet werden kann. Der Entnahmebehälter weist in seiner Verschlussseite vorzugsweise ein Verschlusssystem auf, durch das der Entnahmebehälter hermetisch verschlossen wird, und der Inhalt mit einer Kanüle entnommen werden kann (Figur 2). Alternativ kann der Inhalt des Entnahmebehälters bzw. des Mehrkammermischbehälters durch einen nadelfreien Zugang abgeleitet werden.

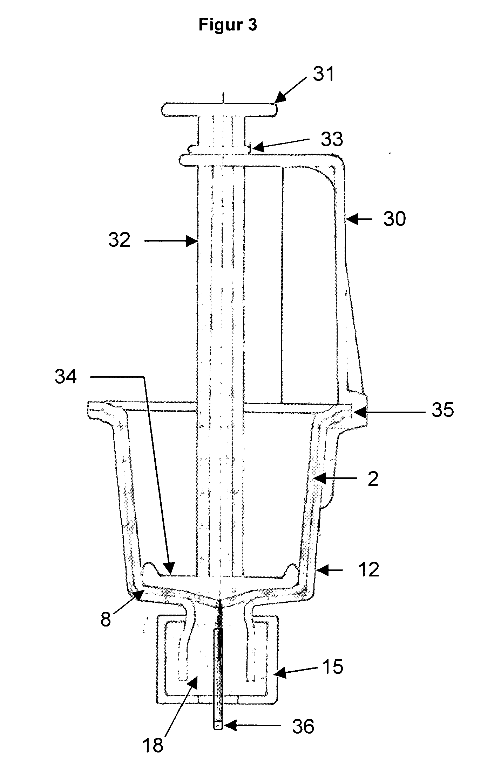

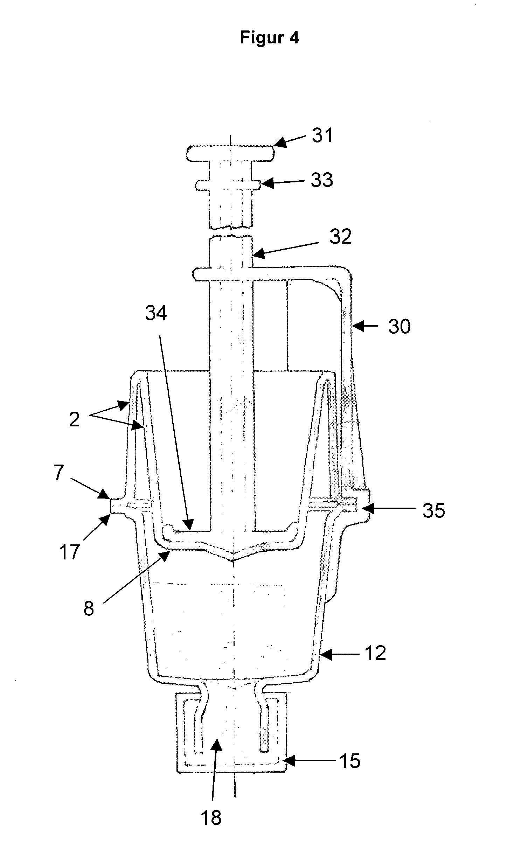

Zur Erfindung gehört weiterhin eine Vorrichtung, in die der Mehrkammermischbehälter eingelegt und durch entsprechende Aussparungen fixiert werden kann. Die Vorrichtung ist halbschalenförmig und weist einen in axialer Richtung verschiebbaren Ausdrückstempel auf. Diese Vorrichtung ist ein karpulenartiges System, das zur Durchmischung der Inhaltsstoffe der Komponentenbehälter verwendet wird (Figur 3). Die Vorrichtung ist vorzugsweise als halbschalenförmige Aufnahme mit einer oder mehreren Zentriernuten in der Verbindungsebene der Komponentenbehälter ausgeführt, die den Mehrkammermischbehälter zentrieren und in seiner Lage stabilisieren. Die Vorrichtung für einen Mehrkammermischbehälter mit einem, zwei oder mehr Zwischenbehältern ist prinzipiell ähnlich aufgebaut, lediglich Länge und Anzahl der vorzugsweise vorhandenen Zentriernuten sind an die jeweilige Konfiguration angepasst. Durch Ausübung von Druck durch den Ausdrückstempel auf den Boden des Stülpbehälters und eine nachfolgende Bewegung des Ausdrückstempels in Richtung Siegelfolie, was auch in Richtung des Entnahmebehälters ist, wird der Stülpbehälter eingedrückt und seine Wandung stülpt sich nach innen. Mit fortschreitender Bewegung des Ausdrückstempels in Richtung Entnahmebehälter durchstößt der Boden des Stülpbehälter die Siegelfolie des Stülpbehälters und danach die Siegelfolie des Entnahmebehälters und die Flüssigkeit aus dem Stülpbehälter kann in den Entnahmebehälter übertreten und den Stoff lösen (Figur 4). Die so frisch zubereitete Lösung kann aus dem Entnahmebehälter durch einen Entnahmebereich wie z.B. einen Stopfen oder ein Septum entnommen werden. Zum vollständigen Entleeren des Mehrkammermischbehälters kann der Stülpbehälter vollständig in den Entnahmebehälter gestülpt werden bis der Boden des Stülpbehälters am Entnahmebereich des Entnahmebehälters anliegt (Figur 3).

### Ausführliche Beschreibung der Erfindung

Überraschend wurde gefunden, dass ein Mehrkammermischbehälter zur getrennten Lagerung und Mischung der enthaltenen Komponenten die gestellte Aufgabe löst, wobei der erfindungsgemäße Mehrkammermischbehälter aus einem Stülpbehälter und einem Entnahmebehälter gefüllt mit einer Flüssigkeit oder einem Feststoff besteht oder diese umfasst, wobei Stülpbehälter und Entnahmebehälter dichtend miteinander verbunden sind, der Entnahmebehälter auf der Oberseite mit einer durchdrückbaren Siegelfolie verschlossen ist und auf der Unterseite einen Entnahmebereich aufweist.

Der erfindungsgemäße Mehrkammermischbehälter besteht in der einfachsten Ausführungsform aus einem Stülpbehälter und einem Entnahmebehälter, wobei der Stülpbehälter immer mit einer Flüssigkeit gefüllt ist. Der Entnahmebehälter enthält vorzugsweise einen Feststoff oder ein Feststoffgemisch, kann aber auch eine weitere Flüssigkeit oder ein Flüssigkeitsgemisch oder eine Lösung von einem oder mehreren Stoffen in einem Lösungsmittel enthalten. Vorzugsweise ist die im Entnahmebehälter enthaltene Komponente länger stabil, wenn diese getrennt von der Flüssigkeit im Stülpbehälter gelagert wird als wenn die im Entnahmebehälter enthaltene Komponente in der Flüssigkeit gelöst ist. Durch Stülpen des Stülpbehälters zumindest teilweise in den Entnahmebehälter erfolgt die Durchtrennung der Unterteilung zwischen beiden Komponentenbehältern und die Komponente enthalten im Entnahmebehälter kann in der Flüssigkeit enthalten in dem Stülpbehälter gelöst werden.

Somit betrifft die vorliegende Erfindung einen Mehrkammermischbehälter umfassend oder bestehend aus dem erfindungsgemäßen Stülpbehälter und einem Entnahmebehälter gefüllt mit einer Flüssigkeit oder einem Feststoff, wobei Stülpbehälter und Entnahmebehälter dichtend miteinander verbunden sind, der Entnahmebehälter auf der Oberseite mit einer durchdrückbaren Siegelfolie verschlossen ist und auf der Unterseite einen Entnahmebereich aufweist. Als Entnahmebereich kann ein Stopfen, Septum, Ventil, Verschluß, Schraubverschluß oder andere Verschlüsse zum Verbinden mit einer Kanüle oder einem Schlauch dienen.

In einer weiteren Ausführungsform besteht der Mehrkammermischbehälter aus dem erfindungsgemäßen Stülpbehälter und dem Entnahmebehälter und einem zwischen Stülpbehälter und Entnahmebehälter befindlichen Zwischenbehälter gefüllt mit einer Flüssigkeit oder einem Feststoff, wobei der Zwischenbehälter an der Oberseite und an der Unterseite mit einer durchdrückbaren Siegelfolie verschlossen ist und der Zwischenbehälter dichtend mit dem Stülpbehälter und dichtend mit dem Entnahmebehälter verbunden ist.

In einer anderen Ausführungsform besteht der Mehrkammermischbehälter aus dem Stülpbehälter und dem Entnahmebehälter und zwei zwischen Stülpbehälter und Entnahmebehälter befindlichen Zwischenbehältern jeweils gefüllt mit einer Flüssigkeit oder einem Feststoff, wobei beide Zwischenbehälter an der Oberseite und an der Unterseite mit einer durchdrückbaren Siegelfolie verschlossen sind und beide Zwischenbehälter dichtend miteinander verbunden sind und der eine Zwischenbehälter dichtend mit dem Stülpbehälter und der andere Zwischenbehälter dichtend mit dem Entnahmebehälter verbunden ist.

Der erfindungsgemäße Mehrkammermischbehälter besteht also immer aus dem Stülpbehälter, der eine Flüssigkeit enthält und dem Entnahmebehälter, der eine feste oder flüssige Komponente enthält und optional einem, zwei oder mehr zwischen Stülpbehälter und Entnahmebehälter liegenden Zwischenbehältern, wobei jeder Komponentenbehälter mit dem anliegenden Komponentenbehälter dichtend verbunden ist, d.h. bevorzugt aseptisch verschweißt oder verklebt ist und die miteinander dichtend verbundenen Flächen der Komponentenbehälter mit einer Siegelfolie verschlossen sind. Dieses Aufbauprinzip ermöglicht die Herstellung und Befüllung und Versiegelung der einzelnen Behälter unter aseptischen Bedingungen, welche dann erst zu dem erfindungsgemäßen Mehrkammermischbehälter zusammengesetzt werden.

Der Stülpbehälter ist dabei durch einen nach innen eindrückbaren Boden, eine gegenüberliegende mit einer Siegelfolie verschlossene Fläche und eine nach innen stülpbare Wandung gekennzeichnet. Der Zwischenbehälter zeichnet sich durch eine stabile, d.h. nicht stülpbare, bis zu einem Teil der Höhe des Behälters stülpbare oder vollständig stülpbare Wandung aus und einer mit einer Siegelfolie verschlossenen Oberseite sowie einer mit einer Siegelfolie verschlossenen Unterseite. Der Entnahmebehälter zeichnet sich durch einen Bereich, den Entnahmebereich, aus, durch den die in dem Mehrkammermischbehälter hergestellte Lösung vorzugsweise durch eine Nadel, Kanüle oder auch nadelfrei entnommen werden kann, eine gegenüberliegende mit einer Siegelfolie verschlossene Seite und eine stabile, d.h. nicht stülpbare Wandung. Zudem weisen alle Komponentenbehälter eine gleichmäßige Geometrie ihrer Querschnittsfläche auf und sind vorzugsweise rund oder oval, so dass ein Einstülpen des Stülpbehälters möglichst bequem erfolgen kann. Zudem weisen die Komponentenbehälter vorzugsweise denselben Innenradius auf, damit diese passend zusammengesetzt werden können und die Verbindungslinie oder der Verbindungsrand aufeinander passen. Der Mehrkammermischbehälter ist somit vorzugsweise zylinderförmig mit einem flachen oder nach innen gewölbten Boden, nämlich dem Boden des Stülpbehälters und einem kopfseitigen Entnahmebereich in Form einer Kappe, Bördelkappe, Schraubkappe, Stopfen oder der gleichen, durch Siegelfolien voneinander getrennten Kompartimenten und einem länglichen vorzugsweise zylinderförmigen Korpus.

Die Komponentenbehälter oder Stülpbehälter und Entnahmebehälter werden so miteinander verbunden, dass die Siegelfolien in kurzem Abstand aufeinander liegen. Zur form-, kraft- und/oder stoffschlüssigen, d.h. dichtenden und aseptischen Verbindung werden die Komponentenbehälter vorzugsweise durch Stecken, Schrauben, Kleben, Einpressen, besonders bevorzugt aber durch Schweißen miteinander verbunden, damit beim Vermischen der Inhalte keine Undichtigkeit auftritt und der aseptische Inhalt weiterhin steril und keimfrei bleibt. Die Verbindung wird unabhängig vom Fügeverfahren aseptisch also unter sterilen Bedingungen durchgeführt, damit keine Keime in den Zwischenraum zwischen den Siegelfolien gelangen. Der optional vorhandene Fügerand gibt der Verbindung einen mechanischen Halt und trägt weiter zum Abdichten der Verbindung bei. Möglich ist auch ein Verschluss des Komponentenbehälters durch vollständiges Verschweißen bzw. Verschmelzen des Komponentenbehältermaterials oder eines Überzugs mit einem flüssigkeits-undurchlässigen Material wie Plastik, einem korrosionsbeständigen Metall oder einem Polymer.

Der durch die Verbindung der Komponentenbehälter entstandene Mehrkammermischbehälter weist vorzugsweise eine zylindrische Form auf, die durch die Form der Komponentenbehälter definiert wird. In Form und Durchmesser entspricht der Mehrkammermischbehälter also Stülpbehälter und Entnahmebehälter als auch dem optionalen Zwischenbehälter.

Die sich gegenüberliegenden Siegelfolien von Stülp- und Entnahmebehälter im Bi-System oder von Stülp-, Zwischen- und Entnahmebehälter im Tri-System, Quad-Sytem oder Multi-System werden zur Vermischung der Inhalte im Sinne der Erfindung von dem Boden des Stülpbehälters oder der Durchdrücknase des Bodens des Stülpbehälters nacheinander durchdrückt. Durch mechanischen Druck auf den Boden des Stülpbehälters wird dabei der Boden oder die Durchdrücknase auf der Bodeninnenseite in Richtung der Siegelfolien gedrückt, die durch den Druck einreißen. Dabei wird zuerst die Siegelfolie des Stülpbehälters und dann die Siegelfolie des Entnahmebehälters oder sofern vorhanden die Siegelfolie des Zwischenbehälters durchstoßen. Durch die entstandenen Öffnungen kommen die Inhalte der Komponentenbehälter miteinander in Kontakt, werden die Flüssigkeiten miteinander gemischt bzw. ein im Entnahmebehälter befindlicher Feststoff in der Flüssigkeit gelöst, welche im Stülpbehälter enthalten war. Der Druck, der für ein Durchdrücken oder Einreißen der Siegelfolien und damit ein Einströmen bzw. Überströmen von Flüssigkeit vom Stülpbehälter in den Entnahmebehälter sorgt, wird durch den auf den Boden des Stülpbehälters ausgeübten mechanischen Druck erzeugt (Figur 3).

Je weiter der Stülpbehälter eingestülpt wird, desto größer wird der Druck im Mehrkammermischbehälter. Dieser erhöhte Druck kann gemindert werden, indem die erhaltene Lösung entweder mit einer Nadel oder auch nadelfrei über den Entnahmebereich des Entnahmebehälters entnommen wird. Bei einer Entnahme mittels Nadel oder Kanüle wird die erhaltene Lösung vorzugsweise in eine Spritze oder eine Volumenmesseinrichtung aufgezogen. Bei einer nadelfreien Entnahme der erhaltenen Lösung erfolgt beispielsweise eine Überführung der Lösung in einen Tropftrichter oder Tropfbeutel worin sich auch schon eine andere Flüssigkeit befinden kann. Soll hingegen die hergestellte Lösung noch nicht entnommen werden, so kann auch der entstehende Druck über eine Nadel, Kanüle oder ein am Entnahmebehälter befindlichen Überdruckventil abgelassen werden.

Der erfindungsgemäße Stülpbehälter ist ein Komponentenbehälter zur Aufnahme einer Flüssigkeit, der stirnseitig mit einer durchdrückbaren Siegelfolie verschlossen ist und dessen Boden in Richtung der Siegelfolie drückbar ist und die Wandung nach innen stülpbar ist.

Der Stülpbehälter wird in seiner äußeren Form durch Boden, Wandung und Stirnseite charakterisiert. Der Begriff der Stülpbarkeit wird durch die Veränderung einer Form definiert. Die stülpbare Wandung wird dabei mit dem Boden des Stülpbehälters nach innen gedrückt, so dass vorzugsweise die Innenseite der gestülpten Wandung an der Innenseite der noch nicht gestülpten Wandung entlang gleitet und der Stülpbehälter während des Stülpvorgangs an Höhe verliert (Figur 4). Besteht der Mehrkammermischbehälter nur aus einem Stülpbehälter und einem Entnahmebehälter so kann bei vollständigem Stülpen des Stülpbehälters die Innenseite der Wandung des Stülpbehälters an der Innenseite der Wandung des Entnahmebehälters anliegen. Weisen Stülpbehälter und Entnahmebehälter eine gleiche Höhe auf, so lässt sich der Stülpbehälter vollständig in den Entnahmebehälter stülpen und der Boden des Stülpbehälters liegt am oder nahe dem Entnahmebereich des Entnahmebehälters (Figur 3).

Der erfindungsgemäße Stülpbehälter ist also dadurch charakterisiert, dass bei entsprechender Wirkung einer Kraft auf seinen Boden die Wandung nach innen gedrückt wird. Mit anderen Worten kann das Stülpen auch als durch Eindrücken bewirkte plastische Verformung des erfindungsgemäßen Stülpbehälters bezeichnet werden. Die stülpbare Wandung ist vorteilhaft, weil dadurch eine Formveränderung durch Eindrücken des Stülpbehälters in den Entnahmebehälter oder in den oder die Zwischenbehälter und den Entnahmebehälter bewirkt wird. Der Druck von außen bedeutet mechanisches Drücken bevorzugt auf die Mitte des Bodens des Stülpbehälters.

Stülpbar gemäß vorliegender Erfindung ist somit die Wandung des Stülpbehälters zumindest so weit, bis die Siegelfolie des Entnahmebehälters durchstoßen wird. Bei einem Mehrkammermischbehälter aus Stülpbehälter und Entnahmebehälter kann der Stülpbehälter vorzugsweise bis zur Verbindungsebene beider Komponentenbehälter gestülpt werden, sofern der Entnahmebehälter die gleiche oder eine größere Höhe hat als der Stülpbehälter. Befindet sich zwischen Stülpbehälter und Entnahmebehälter noch ein Zwischenbehälter, so können die einzelnen Komponentenbehälter dieselbe oder verschiedene Höhen aufweisen. Haben alle drei Komponentenbehälter dieselbe Höhe, dann ist bevorzugt, dass eine Stülpung bis zur mittleren Höhe des Zwischenbehälters erfolgen kann, so dass der Stülpbehälter vollständig in den Entnahmebehälter und der Zwischenbehälter bis zur Hälfte in sich selbst gestülpt werden kann. Besitzen hingegen Entnahmebehälter und Zwischenbehälter zusammen dieselbe Höhe wie der Stülpbehälter, so braucht nur der Stülpbehälter stülpbar ausgestaltet zu sein, damit der Stülpbehälter vollständig in den Entnahmebehälter als auch den Zwischenbehälter gestülpt werden kann. Hat hingegen der Entnahmebehälter dieselbe Höhe wie Stülpbehälter und Zwischenbehälter zusammen, so sollten Stülpbehälter und Zwischenbehälter stülpbar ausgestaltet sein, damit Stülpbehälter als auch Zwischenbehälter in den Entnahmebehälter gestülpt werden können. Natürlich sind auch beliebige andere Höhen der Komponentenbehälter möglich. Vorzugsweise sollte aber ein weitgehendes Stülpen oder Einstülpen möglich sein, wobei der Mehrkammermischbehälter nach vollständigem Stülpen oder Einstülpen ca. 50% seiner Höhe verliert.

Besteht der Mehrkammermischbehälter aus einem Stülpbehälter, einem Entnahmebehälter und zwei Zwischenbehältern, so sind entsprechend auch wieder unterschiedliche Höhen der Komponentenbehälter möglich. Weisen alle vier Komponentenbehälter dieselbe Höhe auf, dann ist bevorzugt, wenn der Stülpbehälter und der am Stülpbehälter angrenzende erste Zwischenbehälter stülpbar sind, so dass bei vollständigem Stülpen oder Einstülpen die Innenseite der Wandung des Stülpbehälters an der Innenseite der Wandung des Entnahmebehälter und die Innenseite der Wandung des ersten Zwischenbehälters an der Innenseite der Wandung des zweiten Zwischenbehälters anliegt. Haben hingegen Entnahmebehälter, erster Zwischenbehälter und zweiter Zwischenbehälter zusammen dieselbe Höhe wie der Stülpbehälter, dann braucht nur der Stülpbehälter stülpbar ausgestaltet zu sein, damit dieser in den Entnahmebehälter und die beiden Zwischenbehälter gestülpt werden kann. Haben Stülpbehälter und die beiden Zwischenbehälter zusammen dieselbe Höhe wie der Entnahmebehälter, dann ist bevorzugt, wenn Stülpbehälter und beide Zwischenbehälter stülpbar ausgestaltet sind, damit diese drei Komponentenbehälter in den Entnahmebehälter gestülpt werden können. Es versteht sich für jeden Fachmann, dass die vier Komponentenbehälter auch andere beliebige Höhen haben können und eine Stülpbarkeit vorzugsweise vom Boden bis zur Mitte des Mehrkammermischbehälters gegeben sein sollte, damit der untere Teil des Mehrkammermischbehälters in den oberen Teil des Mehrkammermischbehälters gestülpt werden kann.

Somit kann es erforderlich sein, einen Komponentenbehälter so auszugestalten, dass nicht seine gesamte Wandung sondern nur bis zu einer bestimmten Höhe die Wandung stülpbar ist.

Die Stülpbarkeit wird in erster Linie durch die Materialbeschaffenheit definiert. Dabei bedeuten besonders feste Materialien keine oder geringe Stülpbarkeit, so dass eine Materialbeschaffenheit bevorzugt ist, die ein leichtes Eindrücken und/oder Umfalten ermöglicht, ohne dass das Material beschädigt wird oder ein zu hoher Kraftaufwand notwendig ist. Jedoch darf das Material nicht so weich sein, dass die Form des flüssigkeitsenthaltenden Stülpbehälters nicht stabil ist oder eine mechanische Beschädigung des Stülpbehälters möglich ist. Der Behälter kann aus einem beliebigen Material bestehen, dass sich zur Aufbewahrung einer Flüssigkeit eignet. Bevorzugte Materialien sind dabei Metall, eine Metalllegierung, Gummi oder ein beliebiges flüssigkeitsundurchlässiges Polymer, wobei Polymere und Gummi bevorzugter sind, und besonders bevorzugt Polymere, und ganz besonders Plastik. Dabei können verschiedene Steifigkeiten des Kunststoffs die Stülpbarkeit beeinflussen. So ist Weichplastik, also besonders biegsamer Kunststoff wie beispielsweise Weich-PVC oder Polyolefine, z.B. Polyethylen, für die Herstellung des stülpbaren Teils besonders bevorzugt, und Hartplastik bevorzugt für den Teil, der nicht gestülpt werden soll. Das Eindrücken bzw. das Einstülpen kann dabei durch sogenannte Rastlinien unterstützt aber auch begrenzt werden. Die Rastlinien, welche die Stülpbarkeit begrenzen sollen, zeichnen sich durch einen Übergang zu einer stärkeren Materialbeschaffenheit aus, die ein weiteres einfaches Einstülpen verhindern, also besonders durch einen Übergang von Weichplastik zu Hartplastik. Vorzugsweise sind die Rastlinien jedoch zur Unterstützung der Stülpbarkeit gedacht und stellen z.B. Verdünnungen in der Wandung dar, so dass ein Umfalten oder Stülpen der Wandung entlang der Rastlinien erleichtert wird. Vorzugsweise kann dadurch ein Abschnitt der Wandung bis hin zur nächsten Rastlinie leichter eingestülpt werden, so dass die Rastlinien wie die beweglichen Verbindungsstellen einer Kette bei einem Kettenfahrzeug wirken und immer ein Glied der Kette bis zur nächsten Rastlinie leichter nach innen gestülpt werden kann. Es ist dabei bevorzugt, wenn die Rastlinien geschlossen ringförmig die Wandung umlaufen. Zudem ist bevorzugt, wenn die Rastlinien in gleichem Abstand zueinander verlaufen.

Das Volumen des erfindungsgemäßen Stülpbehälters, aber auch das Volumen von weiteren erfindungsgemäßen Komponentenbehältern liegt bevorzugt zwischen 0,5 und 20 ml, noch bevorzugter zwischen 1 und 20 ml, bevorzugter zwischen 2 und 18 ml, noch bevorzugter zwischen 3 und 16 ml, noch bevorzugter zwischen 4 und 15 ml, noch bevorzugter zwischen 4 und 14 ml, noch bevorzugter zwischen 5 und 13 ml, noch bevorzugter zwischen 5 und 12 ml und noch bevorzugter zwischen 5 und 11 ml. Die Form des Stülpbehälters ist vorzugsweise zylinderförmig, wobei die Wandung bevorzugt rund ist, der Boden vorzugsweise konkav, also nach innen gewölbt ist und die Stirnseite flach ist. Dabei kann der Boden des Stülpbehälters der Form eines Ausdrückstempels einer Vorrichtung zur Aufnahme des Mehrkammermischbehälters angepasst sein. Dieser Ausdrückstempel hat vorzugsweise eine kegelförmige flache Spitzt, welche beispielsweise in eine entsprechende Aussparung des Bodens des Stülpbehälters eingreift oder an einem entsprechend ausgestalteten Boden des Stülpbehälters anliegt. Diese Anpassung der äußeren Form des Bodens oder zumindest eines zentralen Bereichs des Bodens des Stülpbehälters an die Form der Spitze des Ausdrückstempels ist vorteilhaft, weil dadurch ein Abrutschen des Ausdrückstempels vermieden und eine effiziente mechanische Kraftübertragung gewährleistet werden kann. In einer weiteren bevorzugen Ausführungsform ist der Boden des Stülpbehälters außen flach ausgestaltet. Hier drückt sich dann die Spitze des Ausdrückstempels in den Boden ein, so dass dadurch ein Abrutschen des Ausdrückstempels vermieden wird.

Das Material des Bodens des Stülpbehälters weist vorzugsweise eine höhere Steifigkeit auf als das stülpbare, weiche Material der Wandung, ist also bevorzugt aus Hartplastik. Diese Materialverstärkung ist vorteilhaft, damit durch den vom Ausdrückstempel auf den Boden ausgeübten Druck, der Boden nicht beschädigt wird oder sogar einreißt.

Die im Innenraum des Stülpbehälters liegende Oberfläche des Bodens kann eine Ausgestaltung aufweisen, welche das Durchdrücken der Siegelfolien erleichtert. Erfindungsgemäß kann der Boden des Stülpbehälters eine in Richtung der Siegelfolie ausgebildete und mittig angeordnete Durchdrücknase oder Einwölbung oder Verdickung aufweisen oder der Boden kann konkav geformt sein wie z.B. bei einer Sektflasche. Diese Durchdrücknase, Einwölbung, Verdickung oder konkave Ausgestaltung des Bodens kann aus einem härteren Material sein und/oder entsprechend spitz geformt sein, um ein Durchstoßen der Siegelfolien zu erleichtern.

Diese Durchdrücknase oder Bodenausgestaltung kann durch äußeren Druck auf die Stülpseite, besonders also durch Umstülpen der Wandung axial bewegt werden. Die Durchdrücknase oder Bodenausgestaltung dient dazu, die stirnseitige Siegelfolie des Stülpbehälters zu durchstoßen. Weiterhin dient die Durchdrücknase oder Bodenausgestaltung dazu, die Siegelfolie eines angefügten Komponentenbehälters bzw. die Siegelfolien angefügter Komponentenbehälter zu durchstoßen. Die Durchdrücknase ist vorzugsweise aus demselben Material wie der Stülpbehälter vor allem dann, wenn der Stülpbehälter mit Rastlinien versehen ist, oder aus einem harten Material, welches den einwirkenden Kräften standhalten kann. Ein hartes Material der Durchdrücknase oder Bodenausgestaltung ist vorteilhaft, weil dadurch eine ausreichende Stabilität zum Durchstoßen einer oder mehrerer Siegelfolien gewährleistet wird. Besonders bevorzugtes Material der Durchdrücknase ist dabei Hartplastik. Die Länge der Durchdrücknase oder Bodenausgestaltung ist dabei mindestens so lang, dass sie beim Einstülpen des Bodens bzw. der Wandung die Siegelfolie der Stirnseite und die Siegelfolie des angrenzenden Entnahmebehälters durchstößt. Weiterhin kann sie so lang sein, dass sie den Stülpbehälter auf seiner gesamten Länge durchzieht. Diese Ausführungsform ist vorteilhaft und darum bevorzugt, weil sie sich besonders zum Durchstoßen mehrerer Versiegelungen eignet, wenn mehrere Komponentenbehälter hintereinander angeordnet sind wie z.B. beim Tri-System oder Quad-System.

Die Durchdrücknase oder die Einwölbung oder die Verdickung oder der konkav geformte Boden der Stülpseite des erfindungsgemäßen Stülpbehälters kann Kanäle, Rillen, Wellen, sternförmige Vertiefungen, Hügel, Noppen, Stifte oder andere Oberflächenunebenheiten aufweisen, die ein schnelles Einströmen oder Überströmen der Flüssigkeit aus dem Stülpbehälter in mindestens einen weiteren Komponentenbehälter ermöglichen. Dabei fließt die Flüssigkeit bevorzugt aus dem Stülpbehälter in den mindestens einen weiteren Komponentenbehälter. Dies ist besonders sinnvoll, wenn sich in dem mindestens einen weiteren Komponentenbehälter ein Feststoff befindet. Die Strömung der Flüssigkeit wird besonders durch die axiale Bewegung der innenliegenden Vorrichtung ermöglicht. Es ist jedoch auch möglich, dass, wenn der Entnahmebehälter eine Flüssigkeit enthält, die Flüssigkeit aus einem der weiteren Komponentenbehälter in Richtung Stülpbehälter fließt und sich die Flüssigkeiten auf diese Weise mischen. Das durch die vorgesehenen Kanäle etc. ermöglichte schnelle Überströmen von Flüssigkeit ist vorteilhaft, um besonders bei einem teilweisen oder vollständigen Einstülpen des Stülpbehälters in den stirnseitig angefügten Komponentenbehälter eine schnelle Durchmischung der Flüssigkeiten oder der Lösung oder Suspension des Feststoffs in der Flüssigkeit zu gewährleisten.

Die runde Form der Wandung ist besonders vorteilhaft und besonders bevorzugt, weil sie die Stülpbarkeit am besten ermöglicht. Jedoch kann die Wandung auch durch Kanten in mehrere Teilflächen eingeteilt sein. Dabei ist bevorzugt, wenn die Seitenwandung in mindestens acht Teilflächen aufgeteilt ist, weil eine geringere Zahl von Teilflächen das Stülpen erschwert. Je kleiner der Innenwinkel zwischen den Kanten ist, desto schwerer wird der Stülpvorgang. Sind die Komponentenbehälter nicht rund sondern eckig ausgestaltet, d.h. ist die Querschnittsfläche nicht kreisförmig sondern eckig, dann sollte der Innenwinkel zwischen den Kanten mindestens 135° betragen. Ein kreisförmiger Querschnitt der Komponentenbehälter und damit eine Zylinderform des Mehrkammermischbehälter sind jedoch am meisten bevorzugt, wobei eine ovale Form auch noch bevorzugt ist.

Die Stirnseite des Stülpbehälters ist die dem Boden gegenüberliegende Seite. Die Stirnseite ist mit einer Siegelfolie verschlossen. Der Stülpbehälter wird mit dem Boden nach unter mit der Flüssigkeit befüllt und danach mit der Siegelfolie verschlossen. Die Siegelfolie ist durchdrückbar, das heißt, dass das Durchdrücken, Durchstoßen bzw. Einreißen der Siegelfolie durch die Wirkung einer Kraft möglich ist. Ist die Siegelfolie des Stülpbehälters und des angrenzenden Komponentenbehälters durchtrennt kann die Flüssigkeit aus dem Stülpbehälter in den angrenzenden Komponentenbehälter fließen.

Die Versiegelung des Stülpbehälters durch eine Siegelfolie dient zum sterilen und stoffschlüssigen Verschluss des Behälters. Die Siegelfolie dient zum Versiegeln des Stülpbehälters bzw. ganz allgemein der Komponentenbehälter, welche unter aseptischen Bedingungen befüllt wurden, so dass der Inhalt eines jeden Komponentenbehälters steril und keimfrei bleibt. Die Siegelfolie zum Verschluss der Komponentenbehälter kann z.B. als Verbundfolie vorliegen. Bevorzugte Materialien sind beispielsweise Metalle wie z.B. Aluminiumfolie, des weiteren Kunststoffe, Metall-Kunststoffverbindungen, Polymere oder Kombinationen davon. In einer bevorzugten Ausführungsform ist die Versiegelung als "Weak Seal" ausgeführt, d.h. an einer oder mehrerer Stellen ist das Material der Siegelfolie besonders schwach, so dass es sich leichter durchdrücken lässt, oder es sind Vorstanzungen eingefügt, so dass das Material an diesen Stellen leichter einreißt. Wichtig ist jedoch, dass die Siegelfolie die Vorrichtung vollständig und luftdicht abschließt, um eine sterile Aufbewahrung der Inhalte zu gewährleisten und auch bei gekühlter Lagerung des Mehrkammermischbehälters aufgrund der Materialkontraktion bei Kälte bis vorzugsweise 0°C, weiter bevorzugt -15°C und insbesondere bevorzugt -25°C nicht einreißt. Einem Fachmann sind ausreichend Materialien und Möglichkeiten bekannt, eine solche Siegelfolie zu realisieren. Bevorzugte Materialien sind beispielsweise Metalle wie z.B. Aluminiumfolie, des weiteren Kunststoffe, Metall-Kunststoffverbindungen, Polymere oder Kombinationen davon.

In einer bevorzugten Ausführungsform hat der Stülpbehälter eine Nut an der Außenseite der Wandung oder Einbuchtung in der Außenseite der Wandung. Diese Nut oder Einbuchtung ist vorteilhaft, weil sie in einer Vorrichtung zur Aufnahme des Stülpbehälters, die ein Gegenstück dazu, also eine entsprechende Ausbuchtung oder einen Aufsatz oder einen Zapfen hat, ein Drehen des eingesetzten Stülpbehälters verhindert und eine stabile Position garantiert. Diese Nut oder Einbuchtung befindet sich bevorzugt in Höhe der Stirnseite oder entlang der Kontaktfläche oder Kontaktlinien zweier Komponentenbehälter. Diese Ausführung ist vorteilhaft, um das Einstülpen der Wandung nicht zu behindern. Genauso kann es aber erwünscht sein, dass die Nut oder Einbuchtung das Einstülpen an einer gewissen Stelle begrenzt oder stoppt. Die Anordnung der Nut oder Einbuchtung an einer anderen Höhe der Wandung ist daher ebenfalls bevorzugt. Die Form der Nut oder Einbuchtung ist dabei länglich, und bevorzugt rund, jedoch sind kantige Formen auch möglich. Es ist auch möglich und bevorzugt, wenn der Stülpbehälter statt der Nut oder Einbuchtung eine Ausbuchtung oder einen Aufsatz oder einen Zapfen hat und die besagte Vorrichtung zur Aufnahme des Stülpbehälters eine entsprechende Ausformung aufweist. Diese erfindungsgemäße Vorrichtung zur Aufnahme des Mehrkammermischbehälters wird weiter unten noch ausführlicher beschrieben und umfassend eine halbschalenförmige Aufnahme für den Mehrkammermischbehälter, Haltemittel für den Mehrkammermischbehälter und einen in axialer Richtung beweglichen Ausdrückstempel.

Der erfindungsgemäße Stülpbehälter weist weiterhin bevorzugt einen Fügerand auf. Dieser Fügerand ist vorteilhaft, weil er ein rutschfestes Zusammenfügen mit anderen Behältern ermöglicht und dem Zusammenschluss einen mechanischen Halt vermittelt. Der Fügerand ist dabei an der Stirnseite des Stülpbehälters angebracht. Dabei besteht der Fügerand idealerweise aus demselben Material wie der Stülpbehälter, und zwar wie der nichtstülpbare Teil des Stülpbehälters falls ein solcher Teil vorhanden ist. Zum effizienteren Zusammenfügen von zwei Behältern ist es vorteilhaft, wenn der Fügerand eines Behälters eine Nutung und der andere eine dazu passende Spundung aufweist. Möglich ist auch, dass die Fügeränder einander entsprechende Schraubmöglichkeiten aufweisen. Der Fügerand kann weiterhin bevorzugt auf der Außenseite die besagte Nut, Einbuchtung, Ausbuchtung oder Zapfen aufweisen, die ein Drehen oder Rutschen in einer Vorrichtung zur Aufnahme des Stülpbehälters verhindern soll.

Der erfindungsgemäße Entnahmebehälter wird in seiner äußeren Form durch Stirnseite (Oberseite), Wandung und Unterseite charakterisiert. Die Oberseite des Entnahmebehälters ist wie die Stirnseite (Oberseite) beim Stülpbehälter durch eine durchdrückbare Folie versiegelt, so dass der Inhalt des Entnahmebehälters steril bleibt. Für das Material und die Gestaltung der Siegelfolie gelten dabei dieselben bevorzugten Kriterien wie für die Siegelfolie des Stülpbehälters. Der Entnahmebehälter kann dabei eine Flüssigkeit, eine Lösung oder einen festen Stoff oder ein Feststoffgemisch enthalten. Feste Stoffe sind bevorzugt pulverförmig, weiter bevorzugt lyophilisiert oder gefriergetrocknet. Die Form des Entnahmebehälters ist dabei vorzugsweise der des Stülpbehälters angepasst, d.h. der Durchmesser und die Form der Unterseite entspricht der des Stülpbehälters, wodurch eine dichte, also stoffschlüssige Verbindung gewährleistet werden kann. Es ist also wichtig, dass Stirnseite des Stülpbehälters und Unterseite des Entnahmebehälters beim Bi-System (also ohne Zwischenbehälter) gut zusammenpassen, d.h. bevorzugt denselben Durchmesser haben sollten und vorzugsweise auch dieselbe Wandstärke aufweisen sollten.

Das Material des Entnahmebehälters weist vorzugsweise eine steife Beschaffenheit auf, und ist dabei vorzugsweise so beschaffen wie der nichtstülpbare Teil des Stülpbehälters, falls ein solcher Teil vorhanden ist. Es ist nicht erforderlich, dass der Entnahmebehälter stülpbar ist, vielmehr sollte der Entnahmebehälter nicht stülpbar sein und die Wandung des Entnahmebehälters sollte dem Druck ausgeübt durch den Ausdrückstempel standhalten und nicht deformiert werden. Der Entnahmebehälter ist also vorzugsweise aus Metall oder einem Polymer, bevorzugter aus Plastik, und besonders bevorzugt aus Hartplastik.

Weiterhin ist es vorteilhaft, wenn auch die Wandung des Entnahmebehälters die gleiche Form aufweist wie die des Stülpbehälters, so dass beide Behälter in dieselbe Vorrichtung zur Aufnahme des Mehrkammermischbehälters passen. Weiterhin ist die gleiche Form vorteilhaft, weil dadurch der Stülpbehälter ohne Lücken oder mechanische Behinderung in den Entnahmebehälter gestülpt werden kann (Figur 4). Es ist darum weiterhin vorteilhaft, wenn der Entnahmebehälter der spiegelverkehrten Form des Stülpbehälters entspricht. Der Entnahmebehälter weist also bevorzugt das gleiche Volumen und die gleiche Länge auf wie der Stülpbehälter. Es ist jedoch auch möglich, dass der Entnahmebehälter größere oder kleinere Volumina und Längen aufweist als der Stülpbehälter, wobei der Durchmesser beider Komponentenbehälter gleich oder maximal um 10% verschieden voneinander sein sollte.

Der Entnahmebehälter weist ebenfalls wie der Stülpbehälter vorzugsweise eine Nut, Einbuchtung, Ausbuchtung oder einen Zapfen aufweisen, die ein Drehen oder Verrutschen in einer Vorrichtung zur Aufnahme des Mehrkammermischbehälters verhindern soll. Dabei ist es besonders bevorzugt, wenn die Nuten, Einbuchtungen, Ausbuchtungen oder Zapfen bei den zusammengefügten Behältern entlang der Verbindungsnaht liegen.

Der Entnahmebehälter weist zudem einen Entnahmebereich auf und besitzt bevorzugt einen von einer Nadel durchstechbaren Bereich auf der Unterseite des Entnahmebehälters. Der Entnahmebereich verschließt den Entnahmebehälter hermetisch, so dass der Inhalt steril und keimfrei bleibt und dennoch durch den Entnahmebereich die frisch hergestellte Lösung entnommen werden kann. Bevorzugt ist ein von einer Nadel durchstechbaren Bereich wie z.B. ein Stopfen oder ein Septum, so dass mittels einer Nadel oder einer Kanüle die Lösung entnommen und in eine Spritze aufgezogen oder in einen Behälter oder Beutel überführt werden kann. Der Stopfen kann vorteilhafter Weise durch eine Metallbördelkappe, also ein äußerer Verschluss vorzugsweise aus Aluminiumblech, mit dem Behälter formschlüssig vercrimpt werden. Möglich für Verschlußsysteme dieser Art sind sogenannte Tip-Cap-Verschlüsse. Für einen optimalen Formschluss in Verbindung mit dem Crimpen ist der über den Stopfen hinausgehende Bereich idealerweise mit einem trichterförmigen Hinterschnitt versehen, also einen Rand, um den herum die Bördelkappe vercrimpt werden kann. Prinzipiell besteht auch die Möglichkeit, den Stopfen von der Innenseite des Behälters einzulegen und zu verschweißen, wodurch die Bördelkappe entfallen könnte. Um die Kontaktfläche des Inhalts mit dem Stopfen oder Septum so gering wie möglich zu halten, ist die Variante mit der Bördelkappe vorteilhafter und darum bevorzugt.

Weiterhin ist ein nadelfreier Zugang zum Entnahmebehälter möglich und bevorzugt. Bei einem nadelfreien Zugang wird ein Polymerschlauch mit einem Membranventil durch den Stopfen in den Entnahmebehälter gelegt. Vorstellbar sind dabei nadelfreie Zugänge, wie man sie aus der Infusionstherapie kennt, z.B. beim sogenannten Tropf. Der nadelfreie Zugang ermöglicht eine sichere und bequeme Entnahme des gemischten Inhalts der verbundenen Behälter aus dem Entnahmebehälter. Der Inhalt wird dabei in einen Auffangbehälter wie z.B. einem Tropfbeutel geleitet. Dabei bildet vorzugsweise der Entnahmebehälter mit dem nadelfreien Zugang und dem Auffangbehälter ein geschlossenes, steriles System. Zudem ist es bevorzugt, wenn der Entnahmebehälter ein druckgeregeltes Ausströmventil oder Überdruckventil zum Entweichen von überschüssiger Luft aufweist.

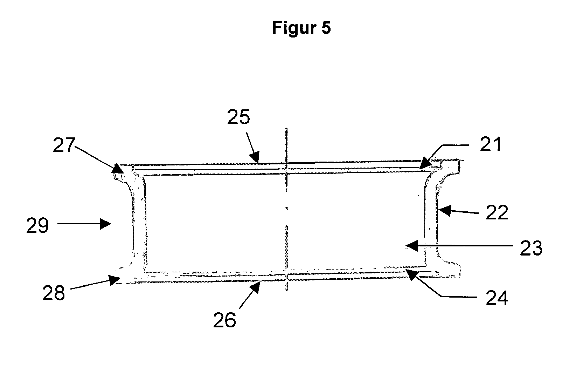

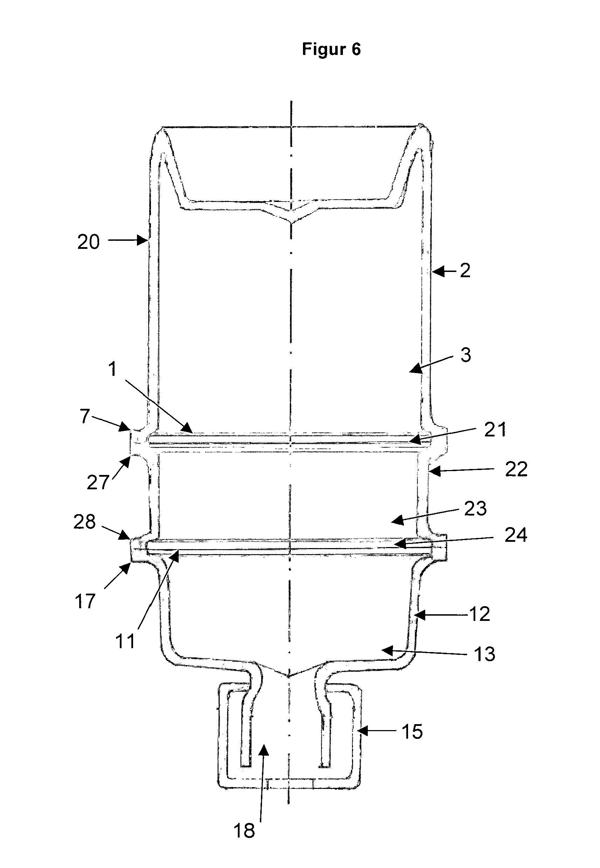

In einer weiteren bevorzugten Ausführungsform besteht der erfindungsgemäße Mehrkammermischbehälter aus einem Stülpbehälter und einem Entnahmebehälter und einem zwischen Stülpbehälter und Entnahmebehälter befindlichen Zwischenbehälter (Figur 5) gefüllt mit einer Flüssigkeit oder einem Feststoff, wobei der Zwischenbehälter an der Oberseite und an der Unterseite mit einer durchdrückbaren Siegelfolie verschlossen ist und der Zwischenbehälter dichtend mit dem Stülpbehälter und dichtend mit dem Entnahmebehälter verbunden ist. In dieser Ausführungsform ist der Mehrkammermischbehälter ein Tri-System (Figur 6).

Der Zwischenbehälter ist also ein weiterer Komponentenbehälter, der eine dritte Komponente zum Mischen bereitstellt. Der Zwischenbehälter wird durch eine Wandung und eine Oberseite als auch eine Unterseite charakterisiert. Sowohl Oberseite als auch Unterseite sind jeweils mit einer durchdrückbaren Siegelfolie verschlossen. Für das Material der Siegelfolie gelten dabei die bevorzugten Kriterien, die bei Stülp- und Entnahmebehälter erwähnt wurden. In dem Mehrkammermischbehälter müssen also insgesamt vier Siegelfolien durchstoßen werden (Stirnseite des Stülpbehälters, Ober- und Unterseite des Zwischenbehälters, Unterseite des Entnahmebehälters), um Kontakt und Durchmischung aller Komponenten zu gewährleisten.

Es ist jedoch auch möglich und bevorzugt, dass die drei Komponentenbehälter so miteinander verbunden sind, dass sich jeweils nur eine Siegelfolie zwischen den entsprechenden Komponenten befindet. Dann würden in dem Mehrkammermischbehälter nur zwei Siegelfolien durchstoßen werden müssen, um Kontakt und Durchmischung der Komponenten zu gewährleisten. Eine Siegelfolie pro Komponentenbehälter wäre dann ausreichend, wenn der Mehrkammermischbehälter sequentiell hergestellt würde, d.h. zuerst der Stülpbehälter mit der Flüssigkeit befüllt und dann mit dem Zwischenbehälter, dessen Unterseite mit einer Siegelfolie verschlossen ist, verbunden wird. Nun würde der Zwischenbehälter befüllt und dann mit dem Entnahmebehälter verbunden, dessen Oberseite mit einer Siegelfolie verschlossen ist. Dabei könnte der Entnahmebehälter bereits gefüllt sein oder erst nach dem Verbinden mit dem Zwischenbehälter durch den offenen Entnahmebereich befüllt werden, wobei dann nach der Befüllung des Entnahmebehälter der Entnahmebereich verschossen wird. Nach diesem sequentiellen Verfahren lassen sich natürlich auch Bi-Systems, Quad-Systems oder generell Multi-Systeme herstellen, wo sich zwischen zwei Komponentenbehältern nur eine Siegelfolie befindet. Dieses sequentielle Herstellungsverfahren vereinfacht den Aufbau des Mehrkammermischbehälters und macht das Durchdrücken einfacher, weil weniger Siegelfolien durchdrückt werden müssen, hat aber den Nachteil, dass die Kombinierbarkeit der Komponentenbehälter miteinander wegfällt.

Die Form des Zwischenbehälters ist dabei vorzugsweise der der anderen Komponentenbehälter angepasst, d.h. entspricht ihnen in Durchmesser und Form, wodurch eine dichte, also stoffschlüssige Verbindung mit den anderen Komponentenbehältern gewährleistet werden kann.

Das Volumen des Zwischenbehälters kann dabei eine beliebige Größe haben, bevorzugt jedoch dem der anderen Komponentenbehälter entsprechen. Bevorzugter ist das Volumen des Zwischenbehälters jedoch kleiner als das von Stülpbehälter oder Entnahmebehälter. Grundsätzlich gibt es jedoch keine Limitierung im Volumen der Komponentenbehälter. Da alle Komponentenbehälter weitgehend denselben Durchmesser aufweisen sollten, wird somit das Volumen des jeweiligen Komponentenbehälters über seine Höhe bestimmt. Da ferner bevorzugt ist, dass nur der Stülpbehälter stülpbar ausgestaltet ist und Entnahmebehälter als auch Zwischenbehälter nicht stülpbar sondern weitgehend formstabil sind, sollte die Höhe des Stülpbehälters ungefähr der Gesamthöhe von Entnahmebehälter und allen Zwischenbehältern entsprechen, damit der Stülpbehälter vollständig in den oder die Zwischenbehälter und den Entnahmebehälter gestülpt werden kann.

Das Material der Wandung des Zwischenbehälters weist vorzugsweise eine steife Beschaffenheit auf, und ist dabei vorzugsweise so beschaffen wie der Entnahmebehälter. Die Wandung des Zwischenbehälters ist also vorzugsweise aus Metall oder einem Polymer, bevorzugter aus Plastik, und besonders bevorzugt aus Hartplastik.

In einer weiteren bevorzugten Ausführungsform kann die Wandung des Zwischenbehälters jedoch auch aus Weichplastik bestehen, also besonders biegsamem Kunststoff wie beispielsweise Weich-PVC oder Polyolefine, z.B. Polyethylen. Durch dieses Material wird eine Stülpbarkeit erreicht. Diese Stülpbarkeit ist vorteilhaft, weil sie ein über das Einstülpen des Stülpbehälters hinausgehendes Stülpen ermöglicht. Das kann z.B. notwendig sein, wenn der Zwischenbehälter zu groß ist, um ein Durchdrücken der unteren Siegelfolie des Zwischenbehälters und des angefügten Entnahmebehälters durch den Durchdrückstempel des Stülpbehälters zu ermöglichen. Der Zwischenbehälter sollte daher immer dann zumindest teilweise oder auch vollständig stülpbar sein, wenn die Höhe des Entnahmebehälters plus die Höhe des Zwischenbehälters größer ist als die Höhe des Stülpbehälters. Ist die Höhe des Entnahmebehälter so groß wie die Höhe des Zwischenbehälters plus die Höhe des Stülpbehälters, dann sollte der Zwischenbehälter auch über seine gesamte Höhe stülpbar sein, damit Zwischenbehälter und Stülpbehälter vollständig in den Entnahmebehälter gestülpt werden können.

In einer weiteren bevorzugten Ausführungsform besteht der erfindungsgemäße Mehrkammermischbehälter aus dem Stülpbehälter und dem Entnahmebehälter und zwei zwischen Stülpbehälter und Entnahmebehälter befindlichen Zwischenbehältern jeweils gefüllt mit einer Flüssigkeit oder einem Feststoff, wobei beide Zwischenbehälter an der Oberseite und an der Unterseite mit einer durchdrückbaren Siegelfolie verschlossen sind und beide Zwischenbehälter dichtend miteinander verbunden sind und der eine Zwischenbehälter dichtend mit dem Stülpbehälter und der andere Zwischenbehälter dichtend mit dem Entnahmebehälter verbunden ist. In dieser Ausführungsform ist der Mehrkammermischbehälter ein Quad-System.

Der zweite Zwischenbehälter ist also ein weiterer Komponentenbehälter zusätzlich zu dem oben beschriebenen (ersten) Zwischenbehälter, der eine vierte Komponente zum Mischen bereitstellt. Der zweite Zwischenbehälter wird durch eine Wandung und zwei durch eine Siegelfolie versiegelte Seiten charakterisiert. Für das Material der Siegelfolie gelten dabei die bevorzugten Kriterien, die bei Stülp-, Entnahme- und erstem Zwischenbehälter erwähnt wurden. In dem Mehrkammermischbehälter müssen also insgesamt sechs Siegelfolien durchstoßen werden (Stirnseite des Stülpbehälters, Ober- und Unterseite des ersten Zwischenbehälters, Ober- und Unterseite des zweiten Zwischenbehälters, Oberseite des Entnahmebehälters), um Kontakt und Durchmischung aller Komponenten zu ermöglichen.

Es ist jedoch auch möglich und bevorzugt, dass die vier Komponentenbehälter so miteinander verbunden sind, dass sich jeweils nur eine Siegelfolie zwischen den entsprechenden Komponenten befindet. Dann würden in dem Mehrkammermischbehälter nur drei Siegelfolien durchstoßen werden müssen, um Kontakt und Durchmischung der Komponenten zu gewährleisten.

Die Form des zweiten Zwischenbehälters ist dabei vorzugsweise der der anderen Komponentenbehälter angepasst, d.h. entspricht ihnen in Durchmesser und Form, wodurch eine dichte, also stoffschlüssige Verbindung gewährleistet werden kann.

Für das Volumen und die Größe des zweiten Zwischenbehälters gelten dabei die gleichen Kriterien wie für den ersten Zwischenbehälter. Dabei können die Zwischenbehälter dieselbe Größe aufweisen, aber auch unterschiedliche groß sein. Das gleiche gilt für das Material der Wandung des zweiten Zwischenbehälters. Auch der zweite Zwischenbehälter kann stülpbar sein ist es jedoch vorzugsweise nicht. Es ist jedoch besonders bevorzugt, wenn von zwei Zwischenbehältern nur einer stülpbar ist. Ist ein Zwischenbehälter stülpbar, so ist dies der mit dem Stülpbehälter verbundene Zwischenbehälter.

In weiteren möglichen bevorzugten Ausführungsformen können auch mehr als zwei Zwischenbehälter in dem Mehrkammermischsystem enthalten sein. In diesem Falle würde man von einem Multi-System sprechen. Dabei ist es bevorzugt, wenn mindestens ein Zwischenbehälter stülpbar ist.

Der oder die Zwischenbehälter weisen wie Entnahmebehälter und Stülpbehälter vorzugsweise eine Nut, Einbuchtung, Ausbuchtung oder einen Zapfen auf, die ein Drehen oder Verrutschen in einer Vorrichtung zur Aufnahme des Mehrkammermischbehälters verhindern soll. Dabei ist es besonders bevorzugt, wenn sich die Nuten, Einbuchtungen, Ausbuchtungen oder Zapfen bei den zusammengefügten Komponentenbehältern entlang der Kontaktflächen der Komponentenbehälter befinden.

Der erfindungsgemäße Mehrkammermischbehälter weist also mehrere Nuten, Einbuchtungen, Ausbuchtungen oder Zapfen auf, die in einer Linie liegen können und mit entsprechenden Zapfen, Ausbuchtungen, Einbuchtungen oder Nuten einer Vorrichtung zur Aufnahme des Mehrkammermischbehälters zusammenpassen, wodurch der Mehrkammermischbehälter in der Vorrichtung rutsch- bzw. drehfest gelagert werden kann.

Alle Zwischenbehälter können bevorzugt einen Fügerand aufweisen. Alle Zwischenbehälter weisen weiterhin vorzugsweise an der Oberseite und/oder der Unterseite, also in der Verbindungsebene der Komponentenbehälter eine Nut oder Einbuchtung oder auch einen Zapfen oder eine Ausbuchtung auf.

Zur Erfindung gehört weiterhin eine Vorrichtung zur Aufnahme des Mehrkammermischbehälters, um die Stülpung durchzuführen und dadurch die gewünschte Lösung frisch zuzubereiten. Diese Vorrichtung umfassend eine halbschalenförmige Aufnahme für den Mehrkammermischbehälter, Haltemittel für den Mehrkammermischbehälter und einen in axialer Richtung beweglichen Ausdrückstempel. Die Vorrichtung ist vorzugsweise ein karpulenartiges System, das zur Durchmischung der Inhaltsstoffe der Komponentenbehälter verwendet wird (Figur 4). Die Vorrichtung ist als halbschalenförmige Aufnahme mit einer oder mehreren Zentriernuten, Einbuchtungen, Zapfen oder Ausbuchtungen als Haltemittel in der Verbindungsebene der Behälter ausgeführt, die den Mehrkammermischbehälter zentriert und in seiner Lage hält. Die Vorrichtung ist dadurch charakterisiert, dass sie den Mehrkammermischbehälter aufnehmen kann, in seiner Lage durch die Haltemittel stabilisiert und weiterhin einen in axialer Richtung beweglichen Ausdrückstempel zur Applikation von äußerem Druck auf den Boden des Stülpbehälters aufweist.

Der in axiale Richtung bewegliche Ausdrückstempel hat vorzugsweise eine Zentrierspitze, die in die vorgesehene Aussparung im Boden des Stülpbehälters passt. Die Zentrierspitze ist vorteilhaft, weil sie eine effiziente Kraftübertragung von Ausdrückstempel auf den Boden des Stülpbehälters ermöglicht und ein Abrutschen des Ausdrückstempels verhindert. Der Ausdrückstempel hat weiterhin vorzugsweise einen Stoppring, um ein unerwünscht weites Eindrücken in die Vorrichtung zu begrenzen.

Die Vorrichtung für ein Tri-, Quad- oder Multi-System ist prinzipiell ähnlich aufgebaut, lediglich Länge der Vorrichtung und Anzahl der Zentriernuten sind an die jeweilige Konfiguration des Mehrkammermischbehälters angepasst.

Zur Erfindung gehört weiterhin eine Methode zur aseptischen Herstellung einer Lösung aus mindestens zwei Komponenten, charakterisiert durch das Einlegen eines Mehrkammermischbehälters in die Vorrichtung zur Aufnahme des Mehrkammermischbehälters, Ausübung von Druck durch den Ausdrückstempel auf den Boden des Stülpbehälters, Drücken des Bodens des Stülpbehälters in Richtung des Entnahmebehälters, wobei sich die Wandung des Stülpbehälters kontinuierlich nach innen stülpt und mit forschreitendem Drücken die Siegelfolien durchdrückt werden und Vermischung der mindestens zwei Komponenten.

Nach der Herstellung der Lösung unter aseptischen Bedingungen erfolgt die Entnahme der hergestellten Lösung durch den Entnahmebereich des Entnahmebehälters mittels Nadel oder auch nadelfrei wie oben geschildert.

Mischbare Komponenten für bevorzugte parenterale Anwendungen, welche in dem Mehrkammermischbehälter vorzugsweise als Feststoff gelagert und kurz vor der Anwendung mit dem im Mehrkammermischbehälter getrennt gelagerten Lösungsmittel vermischt werden können, sind beispielsweise Antibiotika, Analgetika, anti-entzündliche Wirkstoffe, Steroide, antiproliferativen, immunsuppressive, fungizide, zytostatische, antimigrative, antiphlogistische, zytotoxische, antiangiogene oder/und antithrombotische Wirkstoffe, Vitamine, Carotinoide, Schmerzmittel, oder Aminosäuren, oder andere Wirkstoffe. Flüssigkeiten zur Lösung oder Mischung umfassen vorzugsweise aqua bidest., isotonische Natriumchloridlösung, oder verschiedene andere isotonische Salzlösungen.

Im Folgenden wird die vorliegende Erfindung an zwei Beispielen verdeutlicht, welche konkrete Ausführungsformen offenbaren jedoch nicht beschränkend für den Schutzumfang der Erfindung sein sollen. Für einen Fachmann naheliegende Abwandlungen und Änderungen der Erfindung fallen dementsprechend unter den Schutzumfang der Patentansprüche.

**Referenzzeichen**
- 1: Siegelfolie
- 2: Wandung des Stülpbehälters
- 3: Flüssigkeit
- 4: Durchdrücknase oder Einwölbung oder Verdickung
- 5: Stülpbehälter
- 6: Ausformung zur Aufnahme des Ausdrückstempels
- 7: Nut oder Wulst
- 8: Boden des Stülpbehälters
- 9: Stirnseite oder Oberseite des Stülpbehälters
- 10: Rastlinien
- 11: Siegelfolie
- 12: Wandung des Entnahmebehälters
- 13: Feststoff oder Flüssigkeit
- 14: Vertiefung
- 15: Verschluß (Stopfen, Septum, Ventil)
- 16: Entnahmebehälter
- 17: Nut oder Wulst
- 18: Entnahmebereich
- 19: Stirnseite oder Oberseite
- 20: Mehrkammermischbehälter
- 21: Siegelfolie
- 22: Wandung des Zwischenbehälters
- 23: Feststoff oder Flüssigkeit
- 24: Siegelfolie
- 25: Oberseite
- 26: Unterseite
- 27: Nut oder Wulst
- 28: Nut oder Wulst
- 29: Zwischenbehälter
- 30: Vorrichtung zur Aufnahme des Mehrkammermischbehälters
- 31: Griffplatte
- 32: Ausdrückstempel
- 33: Verdickung oder Ring
- 34: Zentrierspitze
- 35: Haltemittel
- 36: Kanüle oder Nadel

### Figurenbeschreibung

### Figur 1

Figur 1 zeigt als Komponentenbehälter einen Stülpbehälter (5), der ca. bis zur Hälfte mit einer Flüssigkeit (3) gefüllt ist. Die Stirnseite oder Oberseite (9) des Stülpbehälters (5) ist mit einer Siegelfolie (1) verschlossen, so dass der Inhalt des Stülpbehälters (5) aseptisch, steril und keimfrei beleibt. Entlang des Randes der Oberseite (9) des Stülpbehälters (5) verläuft eine Wulst oder Nut (7), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Siegelfolie (1) ist derart ausgestaltet, dass sie durch die Durchdrücknase (4) oder Einwölbung (4) oder Verdickung (4) durchstoßen werden kann. Mittig auf dem Boden (8) des Stülpbehälters (5) befindet sich die Durchdrücknase (4) oder Einwölbung (4) oder Verdickung (4). Zudem ist der Boden (8) des Stülpbehälters (5) zylinderförmig nach innen ausgeformt, so dass sich eine Aussparung (6) oder Ausformung (6) zur Aufnahme des Ausdrückstempels (32) ergibt. Der Stülpbehälter (5) weist zudem eine umlaufende Wandung (2) auf, welche aus einem elastischen Polymer besteht. Die Wandung (2) ist zudem mit umlaufenden Rastlinien (10) versehen (nicht gezeigt), so dass ein Stülpen der Wandung (2) von einer bis zur nächsten Rastlinie (10) erleichtert wird. Die Wandung (2) des Stülpbehälters (5) ist so ausgestaltet, dass durch Ausübung von Druck auf den Boden (8) des Stülpbehälters (5) in Richtung der Siegelfolie (1) bzw. in Richtung der Oberseite (9) die Wandung (2) sich am Boden (8) des Stülpbehälters (5) umstülpt und nach innen gedrückt wird, d.h. in die Aussparung oder Ausformung (6), so dass kontinuierlich der Boden (8) des Stülpbehälters (5) in Richtung Siegelfolie (1) wandert. Erreicht der Boden (8) des Stülpbehälters (5) die Siegelfolie (1), dann ist der Stülpbehälter (5) nur noch halb so hoch wie am Anfang, da sich die Hälfte der Außenwandung nun im Inneren befindet.

### Figur 2

Figur 2 zeigt als Komponentenbehälter einen Entnahmebehälter (16), der ca. zu einem Drittel mit einem Feststoff (13) oder einer Flüssigkeit (13) gefüllt ist. Die Oberseite (19) des Entnahmebehälter (16) ist mit einer Siegelfolie (11) verschlossen, so dass der Inhalt des Entnahmebehälter (16) aseptisch, steril und keimfrei beleibt. Die Siegelfolie (11) ist so ausgestaltet, dass sie durch den auf den Boden (8) des Stülpbehälters (5) ausgeübten Druck durchstoßen werden kann. Entlang des Randes der Oberseite (19) des Entnahmebehälters (16) verläuft eine Wulst oder Nut (17), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Siegelfolie (11) ist derart ausgestaltet, dass sie durch die Durchdrücknase (4) oder Einwölbung (4) oder Verdickung (4) des Stülpbehälters (5) durchstoßen werden kann. An der Unterseite weist der Entnahmebehälter (16) einen Entnahmebereich (18) auf, durch den die frisch hergestellte Lösung aus der Komponente des Stülpbehälters (5) und der Komponente des Entnahmebehälters (16) mittels einer Nadel oder Kanüle oder auch nadelfrei entnommen werden kann. Der Entnahmebereich (18) befindet sich in dem Verschluß (15) oder bildet einen Teil des Verschlusses (15). Der Verschluß (15) kann ein Stopfen oder ein Septum sein, welches beispielsweise mittels einer Bördelkappe um den Hals der Unterseite des Entnahmebehälters (16) befestigt ist. Der Stopfen oder das Septum kann aber auch mit dem Material des Entnahmebehälters (16) verklebt oder verschweißt werden. In diesem Fall kann die Bördelkappe entfallen. Der Stopfen oder das Septum können mit einer Nadel oder Kanüle durchstochen werden, so dass die frisch hergestellte Lösung aus dem Mehrkammermischbehälter (20) entnommen werden kann. Unterhalb des Stopfens oder unterhalb des Septums im Inneren des Entnahmebehälters (16) befindet sich der Entnahmebereich (18), nämlich dort, wo die Spitze der Nadel oder der Kanüle endet. Am Boden des Entnahmebehälters (16) kann sich auch eine Vertiefung (14) oder Aussparung (14) befinden. Diese Vertiefung (14) oder Aussparung (14) dient zur Aufnahme der Durchdrücknase (4) oder Einwölbung (4) oder Verdickung (4) des Stülpbehälters (5). Die Lösung kann auch nadelfrei aus dem Mehrkammermischbehälter (20) entnommen werden, wenn der Verschluß (15) als Ventil ausgestaltet ist. Der Entnahmebehälter (16) weist zudem eine umlaufende Wandung (12) auf, welche aus einem festen oder starren Polymer besteht. Der Entnahmebehälter (16) ist nicht stülpbar ausgestaltet. Daher besteht der Entnahmebehälter (16) aus einem festen Material, welches dem Druck auf den Boden (8) des Stülpbehälters (5) standhalten kann, ohne deformiert zu werden.

### Figur 3

Figur 3 zeigt einen Mehrkammermischbehälter (20) bestehend aus einem Stülpbehälter (5) und einem Entnahmebehälter (16), welche dichtend miteinander verbunden sind, eingelegt in eine Vorrichtung (30) zur Aufnahme des Mehrkammermischbehälters (20). Die Vorrichtung (30) umfasst eine halbschalenförmige Aufnahme für den Mehrkammermischbehälter (20) sowie Haltemittel (35) für den Mehrkammermischbehälter (20), um diesen in seiner Lage zu fixieren und einen in axialer Richtung beweglichen Ausdrückstempel (32). Der Ausdrückstemple (32) weist an seiner Hinterseite die Griffplatte (31) auf und eine Verdickung (33) oder einen Ring (33), um die axiale Bewegung des Ausdrückstemples (32) zu begrenzen. An der Spitze des Ausdrückstemples (32) befindet sich die Zentrierspitze (34), welche auf dem Boden (8) des Stülpbehälters (5) aufliegt, um diesen in Richtung Entnahmebehälter (16) bzw. in Richtung des Entnahmebereichs (18) des Entnahmebehälters (16) zu verschieben und dabei den Stülpbehälter (5) immer weiter einzustülpen. Figur 4 zeigt nun den Zustand, wo der Stülpbehälter (5) vollständig in den Entnahmebehälter (16) gestülpt worden ist und der Ausdrückstemples (32) bis zur Verdickung (33) oder bis zum Ring (33) eingedrückt ist. Die Zentrierspitze (34) des Ausdrückstemples (32) ist bis zum Entnahmebereich (18) des Entnahmebehälters (16) vorgeschoben und die Wandung (2) des Stülpbehälters (5) liegt an der Wandung (12) des Entnahmebehälters (16). Die Flüssigkeit (3), welche sich im Stülpbehälter (5) befunden hatte, konnte nach dem Durchdrücken der Siegelfolien (7 und 17) in den Entnahmebehälter (16) fließen und die Komponente (13) des Entnahmebehälters (16) lösen. Diese frisch hergestellte Lösung konnte kontinuierlich während der Fortsetzung des Drückvorgangs aus dem Entnahmebereich (18) und durch den Verschluß (15) des Entnahmebehälters (16) über eine Kanüle (36) ausfließen. Figur 3 zeigt den Zustand, wo der Mehrkammermischbehälter (20) vollständig entleert ist. Der Ausdrückstemple (32) kann nun aus dem eingestülpten Mehrkammermischbehälter (20) herausgezogen, der Mehrkammermischbehälter (20) aus der Vorrichtung (30) entnommen und entsorgt werden.

### Figur 4

Figur 4 zeigt einen Mehrkammermischbehälter (20) bestehend aus einem Stülpbehälter (5) und einem Entnahmebehälter (16), welche dichtend miteinander verbunden sind, eingelegt in eine Vorrichtung (30) zur Aufnahme des Mehrkammermischbehälters (20). Die Vorrichtung (30) umfasst eine halbschalenförmige Aufnahme für den Mehrkammermischbehälter (20) sowie Haltemittel (35) für den Mehrkammermischbehälter (20), um diesen in seiner Lage zu fixieren und einen in axialer Richtung beweglichen Ausdrückstempel (32). Der Ausdrückstemple (32) weist an seiner Hinterseite die Griffplatte (31) auf und eine Verdickung (33) oder einen Ring (33), um die axiale Bewegung des Ausdrückstemples (32) zu begrenzen. An der Spitze des Ausdrückstemples (32) befindet sich die Zentrierspitze (34), welche auf dem Boden (8) des Stülpbehälters (5) aufliegt, um diesen in Richtung Entnahmebehälter (16) bzw. in Richtung des Entnahmebereichs (18) des Entnahmebehälters (16) zu verschieben und dabei den Stülpbehälter (5) immer weiter einzustülpen. Figur 4 zeigt ferner den Zustand, wo der Stülpbehälter (5) etwas mehr als die Hälfte seiner Höhe eingestülpt worden ist, indem auf die Griffplatte (31) des Ausdrückstemples (32) ein Druck ausgeübt wird, der durch die Zentrierspitze (34) des Ausdrückstemples (32) auf den Boden (8) des Stülpbehälters (5) übertragen wird und dadurch den Boden gleichmäßig in axialer Richtung nach vorne schiebt und dabei den Stülpbehälter (5) einstülpt. Damit bei dieser Druckausübung der Mehrkammermischbehälter (20) in der Vorrichtung (30) nicht verrutscht, liegen die miteinander verbundenen (verklebten oder verschweißten) Nuten (7 und 17) in dem Haltemittel (35), welches als Aussparung ausgebildet ist. Ferner ist zu erkennen, dass der Boden (8) des Stülpbehälters (5) mittig eine Durchdrücknase (4) oder Einwölbung (4) oder Verdickung (4) aufweist, welche bereits die Siegelfolie (1) des Stülpbehälters (5) als auch die Siegelfolie (11) des Entnahmebehälters (16) durchdrückt hat, so dass die Flüssigkeit (3) aus dem Stülpbehälter (5) in den Entnahmebehälter (16) übertreten konnte, um die Komponente (13) des Entnahmebehälters (16) zu lösen. Die so entstandene Lösung kann durch oder aus dem Entnahmebereich (18) des Entnahmebehälters (16) mittels einer Nadel oder Kanüle oder auch nadelfrei entnommen werden.

### Figur 5

Figur 5 zeigt als Komponentenbehälter einen Zwischenbehälters (29), der ca. zur Hälfte mit einem Feststoff (23) oder einer Flüssigkeit (23) gefüllt ist. Die Oberseite (25) des Zwischenbehälters (29) ist mit einer Siegelfolie (21) verschlossen, so dass der Inhalt des Zwischenbehälters (29) aseptisch, steril und keimfrei beleibt. Die Siegelfolie (21) ist so ausgestaltet, dass sie durch den auf den Boden (8) des Stülpbehälters (5) ausgeübten Druck durchstoßen werden kann. Entlang des Randes der Oberseite (25) des Zwischenbehälters (29) verläuft eine Wulst oder Nut (27), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Unterseite (26) des Zwischenbehälters (29) ist mit einer Siegelfolie (24) verschlossen, so dass der Inhalt des Zwischenbehälters (29) aseptisch, steril und keimfrei beleibt. Die Siegelfolie (24) ist so ausgestaltet, dass sie durch den auf den Boden (8) des Stülpbehälters (5) ausgeübten Druck durchstoßen werden kann. Entlang des Randes der Unterseite (26) des Zwischenbehälters (29) verläuft eine Wulst oder Nut (28), welche zum dichtenden und/oder stoffschlüssigen Zusammenfügen der Komponentenbehälter vorteilhaft ist und auch als Mittel zum Festhalten in der Vorrichtung (30) zur Aufnahme des Mehrkammermischbehälters (20) dient. Die Siegelfolien (21 und 24) sind derart ausgestaltet, dass sie durch den auf den Boden (8) des Stülpbehälters (5) ausgeübten Druck durchstoßen werden können. Die Wandung (22) des Zwischenbehälters (29) kann starr oder stülpbar oder zum Teil starr und zum Teil stülpbar ausgestaltet sein. Soll der Zwischenbehälter (29) nicht in den Entnahmebehälter (16) gestülpt werden können, dann ist die Wandung (22) aus einem festen Material, welches den Drücken auf den Boden (8) des Stülpbehälters (5) standhalten kann, ohne deformiert zu werden. Soll hingegen der Zwischenbehälter (29) in den Entnahmebehälter (16) stülpbar sein, dann ist die Wandung (22) des Zwischenbehälters (29) flexibel und stülpbar ausgestaltet und kann auch Rastlinien aufweisen. Wird der Zwischenbehälter (29) stülpbar ausgestaltet, so besteht er vorzugsweise aus demselben Material wie der Stülpbehälter (5) und weist auch vorzugsweise die gleiche Art und Ausgestaltung von Rastlinien auf. Soll hingegen der Zwischenbehälter (29) nur teilweise stülpbar sein, so ist seine Wandung (22) bis zu der Höhe stülpbar ausgestaltet, bin wohin der Zwischenbehälter (29) stülpbar sein soll und darüber hinaus besteht die Wandung (22) aus einem festen nicht stülpbaren Material.

### Figur 6

Figur 6 zeigt einen Mehrkammermischbehälter (20) bestehend aus einem Stülpbehälter (5), einem Zwischenbehälter (29) und einem Entnahmebehälter (16), welche dichtend und aseptisch miteinander verbunden sind. Von oben schaut man in die Aussparung (6) oder Ausformung (6) zur Aufnahme des Ausdrückstempels (32) der Vorrichtung (30). Der Boden (8) des Stülpbehälters (5) weist eine Durchdrücknase (4) oder Einwölbung (4) oder Verdickung (4) auf, welche in Richtung Entnahmebehälter (16) zeigt. Der Stülpbehälter (5) ist mit einer Flüssigkeit (3) teilweise gefüllt. Die Wandung (2) des Stülpbehälters (5) weist an der Oberseite (9) eine umlaufende Wulst oder Nut (7) auf, welche stoffschlüssig mit der Wulst oder Nut (27) der Oberseite (25) des Zwischenbehälters (29) verbunden ist. Die stirnseitig angebrachte Siegelfolie (1) des Stülpbehälters (5) liegt über der Siegelfolie (21) des Zwischenbehälters (29). Der Zwischenbehälter (29) ist von der Wandung (22) umgeben, welche am Rand der Unterseite (26) eine weitere Wulst oder Nut (28) aufweist. Zudem befindet sich an der Unterseite (26) eine weitere Siegelfolie (24), wodurch der Inhalt (23) des Zwischenbehälters (29) keimfrei und steril verschlossen ist. Die Wulst oder Nut (28) ist dichtend und stoffschlüssig mit der Wulst oder Nut (17) des Entnahmebehälters (16) verbunden. Unter der Siegelfolie (24) des Zwischenbehälters (29) befindet sich die Siegelfolie (11) des Entnahmebehälters (16). Der Entnahmebehälter (16) ist mit einer Flüssigkeit (13) oder einem Feststoff (13) teilweise gefüllt und von der Wandung (12) umgeben. An der Unterseite weist der Entnahmebehälter (16) einen Verschluß (15) und einen Entnahmebereich (18) auf, durch den oder aus dem die frisch hergestellte Lösung aus der Komponente des Stülpbehälters (5) und der Komponente des Zwischenbehälters (29) und der Komponente des Entnahmebehälters (16) mittels einer Nadel oder Kanüle oder auch nadelfrei entnommen werden kann. Der Mehrkammermischbehälter (20) ist so aufgebaut, dass der Stülpbehälter (5) die gleiche Höhe wie der Zwischenbehälter (29) und der Entnahmebehälter (16) zusammen haben, oder er ist sogar noch etwas größer, so dass der Stülpbehälter (5) vollständig in den Zwischenbehälter (29) und den Entnahmebehälter (16) gestülpt werden kann.

### Beispiele

### Beispiel 1

Ein Stülpbehälter mit einem Gesamtvolumen 10 ml enthaltend 6 ml isotonische Natriumchloridlösung wird mit einem Entnahmebehälter mit einem Gesamtvolumen von 10 ml enthaltend ein lyophilisiertes Bleomycinpräparat in einer Menge von 6 mg an den vorhandenen Fügerändern, die eine Nutung bzw. Spundung aufweisen, unter aseptischen Bedingungen in einer Laminarbox zusammengesteckt und durch Wärmeeinwirkung verschweißt, so dass sich die Siegelfolien der beiden Komponentenbehälter gegenüberliegen. Der so entstandene Mehrkammermischbehälter wird in eine karpulenartige Vorrichtung gelegt, die Vorrichtung geschlossen, und mit dem Ausdrückstempel der Vorrichtung mechanischer Druck auf den Boden des Stülpbehälters ausgeübt. Der Stülpbehälter wird nach und nach eingestülpt, und der innenliegende Durchdrückstempel durchdrückt die beiden Siegelfolien zwischen Stülpbehälter und Entnahmebehälter. Der durch das sich verkleinernde Volumen des Stülpbehälters entstehende Druck führt zu einem schnellen Einströmen der Natriumchloridlösung in den Entnahmebehälter und Auflösen des lyophilisierten Bleomycinderivates. Das Stülpen des Stülpbehälters wird durch umlaufende Rastlinien in der Wandung des Stülpbehälters erleichtert. Die Nut zwischen Stülpbehälter und Entnahmebehälter stoppt den Stülpvorgang. Die überschüssige Luft entweicht durch das druckgeregelte Auslassventil des Entnahmebehälters. Der Stülpvorgang ist beendet, wenn der Stülpbehälter vollständig in den Entnahmebehälter gestülpt wurde. Die Bleomycinlösung wird durch eine Kanüle durch den Stopfen in dem Entnahmebereich auf der Unterseite des Entnahmebehälters entnommen und in einen Tropfbeutel überführt.

### Beispiel 2

Ein Stülpbehälter mit einem Gesamtvolumen von 8 ml enthaltend 5 ml isotonische Natriumchloridlösung wird mit einem Zwischenbehälter mit einem Gesamtvolumen von 4 ml enthaltend 40 mg pulverförmiges Ibuprofen durch Kleben unter sterilen Bedingungen stoffschlüssig zusammengefügt. An die andere Seite des Zwischenbehälters wird ein Entnahmebehälter mit einem Gesamtvolumen von 8 ml durch Kleben unter sterilen Bedingungen stoffschlüssig angefügt, der 500 mg Vitamin C in pulverförmiger Form enthält. Der Stülpbehälter wird eingestülpt, und der innenliegende Ausdrückstempel durchdrückt die beiden Siegelfolien zwischen Stülp- und Zwischenbehälter und nachfolgend die beiden Siegelfolien zwischen Zwischen- und Entnahmebehälter. Der Stülpbehälter hat keine Rastlinien und wird vollständig in das Volumen von Zwischen- und Entnahmebehälter hineingestülpt. Der durch das sich verkleinernde Volumen des Stülpbehälters entstehende Druck führt zu einem schnellen Einströmen der Natriumchloridlösung in den Entnahmebehälter und Auflösen der pulverförmigen Stoffe. Die überschüssige Luft entweicht durch das druckgeregelte Auslassventil des Entnahmebehälters. Die Ibuprofenlösung wird mittels einer Nadel durch den Entnahmebereich in Form eines Septums entnommen und auf eine Spritze aufgezogen.

## Patentansprüche

1. Stülpbehälter zur Aufnahme einer Flüssigkeit, der stirnseitig mit einer durchdrückbaren Siegelfolie verschlossen ist und dessen Boden in Richtung der Siegelfolie drückbar ist und die Wandung nach innen stülpbar ist.

2. Stülpbehälter gemäß Anspruch 1, wobei die Außenseite des Stülpbehälters in Höhe der Siegelfolie eine Nut oder Einbuchtung aufweist.

3. Stülpbehälter gemäß Anspruch 1 oder 2, wobei der Boden des Stülpbehälters eine in Richtung der Siegelfolie ausgebildete und mittig angeordnete Durchdrücknase oder Einwölbung oder Verdickung aufweist oder der Boden konkav geformt ist.

4. Stülpbehälter gemäß Anspruch 3, wobei die Durchdrücknase oder die Einwölbung oder die Verdickung oder der konkav geformte Boden Kanäle, Rillen, Wellen, sternförmige Vertiefungen, Hügel, Noppen, Stifte oder andere Oberflächenunebenheiten aufweist.

5. Stülpbehälter gemäß einem der vorherigen Ansprüche, wobei die stülpbare Wandung mit umlaufenden Rastlinien versehen ist.

6. Mehrkammermischbehälter umfassend oder bestehend aus dem Stülpbehälter gemäß einem der Ansprüche 1 - 5 und einem Entnahmebehälter gefüllt mit einer Flüssigkeit oder einem Feststoff, wobei Stülpbehälter und Entnahmebehälter dichtend miteinander verbunden sind, der Entnahmebehälter auf der Oberseite mit einer durchdrückbaren Siegelfolie verschlossen ist und auf der Unterseite einen Entnahmebereich aufweist.

7. Mehrkammermischbehälter gemäß Anspruch 6 bestehend aus dem Stülpbehälter und dem Entnahmebehälter und einem zwischen Stülpbehälter und Entnahmebehälter befindlichen Zwischenbehälter gefüllt mit einer Flüssigkeit oder einem Feststoff, wobei der Zwischenbehälter an der Oberseite und an der Unterseite mit einer durchdrückbaren Siegelfolie verschlossen ist und der Zwischenbehälter dichtend mit dem Stülpbehälter und dichtend mit dem Entnahmebehälter verbunden ist.

8. Mehrkammermischbehälter gemäß Anspruch 6 bestehend aus dem Stülpbehälter und dem Entnahmebehälter und zwei zwischen Stülpbehälter und Entnahmebehälter befindlichen Zwischenbehältern jeweils gefüllt mit einer Flüssigkeit oder einem Feststoff, wobei beide Zwischenbehälter an der Oberseite und an der Unterseite mit einer durchdrückbaren Siegelfolie verschlossen sind und beide Zwischenbehälter dichtend miteinander verbunden sind und der eine Zwischenbehälter dichtend mit dem Stülpbehälter und der andere Zwischenbehälter dichtend mit dem Entnahmebehälter verbunden ist.

9. Mehrkammermischbehälter gemäß Anspruch 6, 7 oder 8, wobei der Mehrkammermischbehälter gemäß Anspruch 6 außen entlang der Verbindungslinie zwischen Stülpbehälter und Entnahmebehälter eine Nut aufweist oder der Mehrkammermischbehälter gemäß Anspruch 7 außen entlang der Verbindungslinie zwischen Stülpbehälter und Zwischenbehälter und/oder zwischen Zwischenbehälter und Entnahmebehälter eine Nut aufweist oder der Mehrkammermischbehälter gemäß Anspruch 8 außen entlang der Verbindungslinie zwischen Stülpbehälter und dem einen Zwischenbehälter und/oder zwischen den beiden Zwischenbehältern und/oder zwischen dem anderen Zwischenbehälter und Entnahmebehälter eine Nut aufweist.

10. Vorrichtung zur Aufnahme des Mehrkammermischbehälters gemäß einem der Ansprüche 6 - 9 umfassend eine halbschalenförmige Aufnahme für den Mehrkammermischbehälter, Haltemittel für den Mehrkammermischbehälter und einen in axialer Richtung beweglichen Ausdrückstempel.

11. Vorrichtung gemäß Anspruch 10, wobei der Ausdrückstempel eine Zentrierspitze aufweist.

12. Vorrichtung gemäß Anspruch 10 oder 11, wobei als Haltemittel Aussparungen in der halbschalenförmigen Aufnahme zur Aufnahme der Nut oder der Nuten des Mehrkammermischbehälters vorgesehen sind.

13. Methode zur aseptischen Herstellung einer Lösung aus mindestens zwei Komponenten, charakterisiert durch
das Einlegen eines Mehrkammermischbehälters gemäß einem der Ansprüche 6 - 9 in eine Vorrichtung gemäß einem der Ansprüche 10 - 13,
Ausübung von Druck durch den Ausdrückstempel auf den Boden des Stülpbehälters,
Drücken des Bodens des Stülpbehälters in Richtung des Entnahmebehälters, wobei sich die Wandung des Stülpbehälters kontinuierlich nach innen stülpt und mit forschreitendem Drücken die Siegelfolien durchdrückt werden und Vermischung der mindestens zwei Komponenten.
